Europäisches Patentamt

European Patent Office

Office européen des brevets

(11) Veröffentlichungsnummer: **0 486 777 A1**

# EUROPÄISCHE PATENTANMELDUNG

(21) Anmeldenummer: **91114786.6**

(22) Anmeldetag: **03.09.91**

(51) Int. Cl.5: **C07C 45/38**, B01J 23/50

(30) Priorität: **20.11.90 DE 4036897**

(43) Veröffentlichungstag der Anmeldung:
**27.05.92 Patentblatt 92/22**

(84) Benannte Vertragsstaaten:
**AT BE CH DE ES FR GB GR IT LI NL**

(71) Anmelder: **Degussa Aktiengesellschaft**
**Weissfrauenstrasse 9**
**W-6000 Frankfurt am Main 1(DE)**

(72) Erfinder: **Claus, Gottfried, Dr.**
**Kohlgrubenweg 11**
**W-5760 Arnsberg(DE)**
Erfinder: **Hausselt, Jürgen, Dr. Dipl.-Phys.**
**Emeserweg 11**
**W-8755 Alzenau-Michelbach(DE)**
Erfinder: **Weise, Wolfgang, Dr. Dipl.-Ing.**
**Eckenheimer Landstrasse 146**
**W-6000 Frankfurt am Main(DE)**
Erfinder: **Wolmer, Roger**
**Harmoniestrasse 8**
**W-6450 Hanau 7(DE)**

(54) Silberkatalysator für die Formaldehydsynthese und Verfahren zu seiner Herstellung.

(57) Zur Formaldehydsynthese aus Methanol werden Silberkatalysatoren mit hohen Standzeiten benötigt, die auch bei Reaktionstemperaturen von 750° C nicht zusammensintern. Solche Katalysatoren enthalten neben Silber 0 bis 5 Gew.% eines Edelmetalls und 0,01 bis 5 Gew.% eines unter reduzierenden Reaktionsbedingungen thermodynamisch stabilen Oxids, das im Silber feindispers und homogen verteilt ist.

Rank Xerox (UK) Business Services

EP 0 486 777 A1

Die Erfindung betrifft einen Silberkatalysator in Teilchenform für die Formaldehydsynthese. Außerdem werden Verfahren zu seiner Herstellung angegeben.

Zur Herstellung von Formaldehyd wird in Gegenwart eines Silberkatalysators Methanol mit Luft bei höheren Temperaturen umgesetzt. Als Katalysator wird normalerweise eine Schüttung aus Kristallsilber verwendet, über die das Reaktionsgas geleitet wird. Das Kristallsilber besteht aus Teilchen mit stark unregelmäßiger Oberflächenmorphologie, deren Agglomerate im Teilchengrößenbereich von 0,1-10 mm liegen. Die Reaktionstemperatur liegt üblicherweise bei 550-700°C.

Die Umsetzung von Methanol zu Formaldehyd erfolgt über eine exotherme Reaktion (1) und eine endotherme Reaktion (2).

$$CH_3OH + 1/2\ O_2 \longrightarrow HCHO + H_2O \qquad (1)$$

$$CH_3OH \xrightarrow{\text{Temp.}} HCHO + H_2 \qquad (2)$$

Das Reaktionsgemisch enthält Sauerstoff und Methanol und wird so eingestellt, daß es sich oberhalb des Explosionsbereiches befindet.

Zur Verbesserung der Wirtschaftlichkeit ist es wünschenswert, den erforderlichen Überschuß an Methanol durch Inertgas oder Wasserdampf zu ersetzen.

Aus dieser gewünschten Betriebsweise resultieren höhere Reaktionstemperaturen am Katalysatormaterial im Bereich von 650 bis 750° C. Bei Verwendung von Kristallsilber als Katalysator führt dies zu einem Zusammensintern der Silberkristalle. Dadurch wird die Strömungsgeschwindigkeit des Reaktionsgases vermindert, was zu einem ansteigenden Druck im Reaktor führt. Daraus resultiert eine schnelle Minderung des Durchsatzes und die Abnahme der Anlagenleistung. Das Zusammensintern des Kristallsilbers beschränkt den Einsatzbereich des Katalysators auf relativ niedrige Reaktionstemperaturen, wenn ein häufiges Auswechseln des Katalysators vermieden werden soll.

Auch bei normaler Betriebsweise sintert der Katalysator mit zunehmender Einsatzzeit zusammen, so daß auch hier die Lebensdauer des Katalysators durch das Zusammensintern begrenzt wird.

Neben der Verwendung von Silberkristallen als Katalysator ist der Zusatz von Palladium zu Silber bekannt. In der GB-PS 2,121,787 wird ein Silber-Katalysator beschrieben, der bis zu 5 % Palladium enthält.

Palladium führt zu einer Schmelzpunkterhöhung des Silberkatalysators, was eine geringere Tendenz zum Zusammensintern bewirkt.

Eine deutliche Schmelzpunkterhöhung wird aber erst bei höheren Palladium-Gehalten erzielt. Aufgrund des hohen Preises von Palladium wird die Wirtschaftlichkeit vermindert, zudem bewirken zu hohe Palladium-Gehalte im Silber eine reduzierte Selektivität im Vergleich zu reinem Kristallsilber.

Es war Aufgabe der vorliegenden Erfindung, einen Silberkatalysator in Teilchenform für die Formaldehydsynthese zu entwickeln, der auch bei Reaktionstemperaturen von 650 bis 750° C langzeitig betrieben werden kann, ohne daß er zusammensintert und dadurch die Ausbeute und die Standzeit des Katalysators vermindert werden.

Diese Aufgabe wird erfindungsgemäß dadurch gelöst, daß der Katalysator aus 90 bis 99,99 Gew.% Silber, 0 bis 5 Gew.% Palladium, Gold, Platin, Rhenium, Rhodium, Ruthenium und/oder Iridium und 0,01 bis 5 Gew.% eines oder mehrerer unter reduzierenden Reaktionsbedingungen thermodynamisch stabilen, im Silber feindispers und homogen verteilten Oxiden besteht.

Vorzugsweise verwendet man als Oxid eine oder mehrere der Verbindungen Magnesiumoxid, Kalziumoxid, Bariumoxid, Ceroxid, Titandioxid, Zirkoniumdioxid, Hafniumoxid, Aluminiumoxid, Siliziumdioxid und Yttriumoxid. Besonders bewährt haben sich Magnesiumoxid, Zirkoniumoxid, Siliziumdioxid, Yttriumoxid und insbesondere Aluminiumoxid.

Vorzugsweise stellt man diese Katalysatoren dadurch her, daß Silberpulver bzw. Silberlegierungspulver mit den entsprechenden Mengen Oxidpulver gemischt, kaltisostatisch gepreßt und gesintert werden. Anschließend werden die gepressten und gesinterten Formkörper spanabhebend bearbeitet, so daß Späne entstehen, die als Katalysator eingesetzt werden können. Das Sintern erfolgt vorteilhafterweise bei 650 bis 750° C an Luft.

Ein gleichwertiges Herstellungsverfahren besteht darin, daß eine Silberlegierung mit den entsprechenden Mengen eines oder mehrerer der oxidbildenden Elemente geschmolzen und zu Pulver verdüst wird, das man anschließend bei Sauerstoffpartialdrucken von 0,1 bis 1 MPa innerlich oxidiert.

Die Dispersion von thermodynamisch stabilen Oxiden im Silber hat den überraschenden Vorteil, daß die

2

katalytische Wirkung des Silberkatalysators auch bei Gehalten bis 5 Gew.% nicht beeinträchtigt wird. Der erfindungsgemäße Katalysator sintert selbst bei hohen Reaktionstemperaturen von 750° C nicht zusammen und besitzt daher eine hohe Standzeit.

Die erfindungsgemäßen Katalysatoren können auch zu Drähten und Netzen verarbeitet werden.

Folgende Beispiele sollen die Erfindung näher erläutern:

1. Hergestellt wurde ein Katalysator aus Silber mit 2 Gew.% Aluminiumoxid, indem Silberpulver mit $Al_2O_3$-Pulver gemischt, kaltisostatisch gepreßt und bei 700° C an Luft gesintert wurde. Der so erzeugte Bolzen wurde durch Drehen zu Spänen mit Längen von ca. 0,5 bis 10 mm verarbeitet. Die Katalysatorspäne wurden in eine Produktionsanlage eingebaut und unter verschiedenen Betriebsbedingungen getestet. Die Menge des Katalysators betrug 16 kg.

| Betriebsweise 1 | |
|---|---|
| Kontakttemperatur | 710 - 720° C |
| Belastung je m² | 1200 m³ |
| Ausbeute | 90,4 % |
| Ameisensäure % | 0,0076. |

| Betriebsweise 2 | |
|---|---|
| Kontakttemperatur | 690° C |
| Belastung je m² | 1200 m³ |
| Ausbeute | 89,7 % |
| Ameisensäure % | 0,0106. |

| Betriebsweise 3 | |
|---|---|
| Kontakttemperatur | 570° C |
| Belastung je m² | 1200 m³ |
| Ausbeute | 89,0 % |
| Ameisensäure % | 0,0130. |

Für die Beurteilung der erfindungsgemäßen Katalysatoren wurde ein Vergleich mit einem Standard-Katalysator aus Kristallsilber herangezogen.

Dieser wurde unter folgenden Betriebsbedingungen getestet. Die Menge des Standardkatalysators betrug dabei 35 kg:

| Kontakttemperatur | 620° C |
|---|---|
| Belastung je m² | 1100 m³ |
| Ausbeute | 90,2 % |
| Ameisensäure % | 0,066. |

Der erfindungsgemäße Katalysator sintert auch bei hohen Reaktionstemperaturen von 720 bis 750° C nicht zusammen und zeigt dadurch die vierfache Standzeit im Vergleich zum Standard-Katalysator. Dabei bleiben die Ausbeuten gleich gut. Die Bildung der unerwünschten Ameisensäure erfolgt bei dem erfindungsgemäßen Katalysator in vernachlässigbaren Mengen.

2. Hergestellt und getestet wurde ein Katalysator der Zusammensetzung 99 % Silber / 1 % Aluminiumoxid. Zunächst wurde eine entsprechende Silber-Aluminium-Legierung unter Vakuum erschmolzen und zu Pulver verdüst. Die Korngröße des verdüsten Pulvers lag größenordnungsmäßig bei 1,0 - 3,0 mm Durchmesser. Das Pulver wurde anschließend bei 800° C unter einem Sauerstoffpartialdruck von 0,4 MPa oxidiert. Der Katalysator wurde in eine Produktionsanlage eingebaut und getestet. Er sinterte nicht zusammen und wies eine weitaus höhere Lebensdauer auf als der Standard-Katalysator.

3. Weitere Katalysatoren wurden in Versuchsanlagen getestet und zeigten positive Ergebnisse. Sie führten zu guten Formaldehydausbeuten und sinterten nicht zusammen. Diese Katalysatoren sind in der nachfolgenden Tabelle dargestellt.

| Katalysator | Zusammensetzung | |
|---|---|---|
| | [Gew.% Ag] | [Gew.% Oxid] |
| Silber-Magnesiumoxid | 99,5<br>99,0<br>96.0 | 0,5<br>1,0<br>4,0 |
| Silber-Zirkoniumoxid | 99,5<br>99,0<br>96,0 | 0,5<br>1,0<br>4,0 |
| Silber-Siliziumdioxid | 99,5<br>99,0<br>96,0 | 0,5<br>1,0<br>4,0 |
| Silber-Yttriumoxid | 99,5<br>99,0<br>96,0 | 0,5<br>1,0<br>4,0 |
| Silber-Aluminiumoxid | 99,8<br>99,5<br>96,0 | 0,2<br>0,5<br>4,0 |

**Patentansprüche**

1. Silberkatalysator in Teilchenform für die Formaldehydsynthese,
   dadurch gekennzeichnet,
   daß er aus 90 bis 99,99 Gew.% Silber, 0 bis 5 Gew.% Palladium, Gold, Platin, Rhenium, Rhodium, Ruthenium und/oder Iridium und 0,01 bis 5 Gew.% eines oder mehrerer unter reduzierenden Reaktionsbedingungen thermodynamisch stabilen, im Silber feindispers und homogen verteilten Oxiden besteht.

2. Silberkatalysator nach Anspruch 1,
   dadurch gekennzeichnet,
   daß er als Metalloxid Magnesiumoxid, Kalziumoxid, Bariumoxid, Ceroxid, Titandioxid, Zirkoniumdioxid, Hafniumoxid, Aluminiumoxid, Siliziumdioxid und/oder Yttriumoxid enthält.

3. Silberkatalysator nach Anspruch 1,
   dadurch gekennzeichnet,
   daß er als Oxid Magnesiumoxid, Siliziumdioxid, Zirkoniumoxid, Yttriumoxid und/oder Aluminiumoxid, enthält.

4. Verfahren zur Herstellung von Silberkatalysatoren nach Anspruch 1 bis 3,
   dadurch gekennzeichnet,
   daß Silberpulver bzw. Silberlegierungspulver mit entsprechenden Mengen Oxidpulver gemischt, kaltisostatisch gepresst, gesintert und der Formkörper zu Spänen spanabhend bearbeitet wird.

5. Verfahren zur Herstellung von Silberkatalysatoren nach Anspruch 1 bis 3,
   dadurch gekennzeichnet,
   daß Silberlegierungen mit den entsprechenden Mengen der oxidbildenden Metalle erschmolzen, zu Pulver verdüst und die Pulverteilchen bei Sauerstoffpartialdrucken von 0, 1 bis 1 MPa innerlich oxidiert werden.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | US-A-2 005 645 (H. A. BOND ET AL.) <br> * Seite 2, Zeile 10 - Zeile 22 * <br> --- | 1-4 | C07C45/38 <br> B01J23/50 |
| X | CHEMICAL ABSTRACTS, vol. 101, no. 4, <br> Juli 1984, Columbus, Ohio, US; <br> abstract no. 29135N, <br> SZUSTAKOWSKI ET AL.: 'MODIFIED SILVER CATALYSTS' <br> * Zusammenfassung * <br> & PL-A-122 783 <br> --- | 1-3 | |
| X | CHEMICAL ABSTRACTS, vol. 93, no. 17, <br> Oktober 1980, Columbus, Ohio, US; <br> abstract no. 167537N, <br> LAZAROV ET AL.: 'OXIDATION OF METHANOL ON THE <br> SURFACES OF MIXED OXIDE-SILVER CATALYSTS,BASED <br> ON SILVER ALLOYS' <br> Seite 615 ; <br> * Zusammenfassung * <br> & GETEROG. KATAL. <br> Bd. 4, Nr. 2, 1979, <br> Seiten 145 - 150; 'AG ALLOYS WITH Y,Mg, AND Al <br> CATALYSED THE OXIDATION OF MeOH TO HCHO.' <br> --- | 1-3 | |
| | | | RECHERCHIERTE SACHGEBIETE (Int. Cl.5 ) |
| X | FR-A-2 306 011 (S.A.E.S. GETTERS) <br> * Seite 2, Zeile 23 - Zeile 29 * <br> * Seite 4, Zeile 11 - Zeile 13 * <br> --- | 1,2 | C07C <br> B01J |
| X | US-A-1 937 381 (H. A.  BOND ET AL.) <br> * Seite 2, Zeile 94 - Zeile 103 * <br> --- | 1,2 | |
| A,D | GB-A-2 121 787 (MITSUBISHI GAS) <br> * Seite 3, Zeile 13 - Zeile 16 * <br><br> ----- | 1 | |

Der vorliegende Recherchenbericht wurde für alle Patentansprüche erstellt

| Recherchenort | Abschlußdatum der Recherche | Prüfer |
|---|---|---|
| DEN HAAG | 19 NOVEMBER 1991 | KERRES P.M.G. |